(19) Europäisches Patentamt
European Patent Office
Office européen des brevets

(11) Publication number: **0 473 349 B1**

(12) # EUROPEAN PATENT SPECIFICATION

(45) Date of publication of patent specification: **24.05.95**

(51) Int. Cl.$^6$: **A61K 7/06**, A61K 7/08

(21) Application number: **91307643.6**

(22) Date of filing: **20.08.91**

(54) **Hair cosmetic composition.**

(30) Priority: **28.08.90 JP 225523/90**

(43) Date of publication of application:
**04.03.92 Bulletin 92/10**

(45) Publication of the grant of the patent:
**24.05.95 Bulletin 95/21**

(84) Designated Contracting States:
**DE ES FR GB IT**

(56) References cited:
**EP-A- 0 104 653**
**WO-A-89/01771**
**FR-A- 2 203 806**
**GB-A- 1 155 712**

(73) Proprietor: **Kao Corporation**
**1-14-10, Nihonbashikayaba-cho,**
**Chuo-ku**
**Tokyo (JP)**

(72) Inventor: **Tashiro, Kazuhiro**
**1-4-3, Tomiokanishi**
**Kanazawa-ku,**
**Kanagawa (JP)**
Inventor: **Yahagi, Kazuyuki**
**6-1-6-749, Oojima**
**Koto-ku,**
**Tokyo (JP)**

(74) Representative: **Daley, Michael John et al**
**F.J. CLEVELAND & COMPANY**
**40/43 Chancery Lane**
**London, WC2A 1JO (GB)**

EP 0 473 349 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a hair cosmetic composition and more particularly to a so-called after-shampoo composition such as hair rinsing composition, hair conditioning composition or a treatment composition, which have properties of extendibility, smoothness, mildness to the skin and enhanced emulsion stability.

Conventionally, hair cosmetic compositions containing a cationic surfactant are known to impart the hair softness and smoothness when applied to the shampooed hair. Such compositions are generally used as a hair rinse, hair treatment or the like. They are generally applied directly to the wet hair after shampooing and then rinsed off. In such a situation, they must have a property of good extendibility, because, if not, they cannot reach the entire surfaces of the hair fibers, which causes partial stiffness of the hair or the irregularity of the treatment effect. In order to meet this requirement, oils have conventionally been incorporated, which include higher alcohols, glycerides, liquid paraffin and esters.

To stably emulsify or disperse these oils which are incorporated in such an amount that will achieve the above-mentioned effects, surfactants having a higher hydrophilicity are used. Since anionic and nonionic surfactants significantly reduce the rinsing effect and treatment effect of the composition, conventional practice used abundant cationic surfactants blended thereto. High concentration of cationic surfactants, however, may cause irritation of the skin and the scalp, and therefore, efforts are made to reduce the amount of the cationic surfactants which are to be incorporated.

FR-A-2203806 discloses cosmetic compositions comprising N-substituted long chain aliphatic carbonyl amino acid diesters. In some of the examples of FR-A-2203806, polyethylene glycol is used. FR-A-2203806 is, however, silent on the molecular weight of the polyethylene glycol used.

GB-A-1155712 discloses cosmetic compositions comprising a mixture of certain specified polyhydroxylated ethers. Example 12 of GB-A-1155712 discloses a cosmetic composition comprising such a polyhydroxylated ether and polyethylene glycol of molecular weight 5,000,000. Example 12 of GB-A-1155712 does not include an oil or fat.

Japanese Patent Publication No. 1363/1987 discloses a hair rinse composition containing a quaternary ammonium salt, a silicone derivative and a polyethylene glycol having an average molecular weight of 200 - 100,000.

This composition has an enhanced dispersion stability and good texture of the wet hair due to the synergestic effect of a cationic surfactant and a polyethylene glycol. The achieved texture of the wet hair, however, still has room to be improved, and moreover, irritation caused by the cationic surfactant is still present.

Japanese Patent Application Laid-Open Nos 66638/1987 and 154529/1987 disclose a hair conditioning lotion composition which is capable of softening the hair by the use of a polyethylene glycol having an average molecular weight of 10,000 or less. This composition, however, provides insufficient smoothness when applied to the hair.

Japanese Patent Application Laid-Open No. 113312/1989 discloses a split-hair fixing composition which contains a polyethylene glycol which is solid at room temperature, xanthan gum and casein. This composition, however, does not possess functions which are required by after-shampoo compositions, namely properties of imparting the hair smoothness and softness.

Japanese Patent Application Laid-Open No. 161210/1985 discloses a cosmetic composition for massaging which contains a polyethylene oxide and a polyvalent alcohol. This composition is described to have a spinnability achieved by the incorporation of a polyethylene oxide, and good stability, too. Howver, no mention is given to after-shampoo compositions such as hair rinses and hair treatments.

Accordingly, a hair cosmetic composition meeting the requirements of good extendibility upon application, less greasiness, excellent smoothness, mildness to the skin and enhanced stability of emulsion is still required.

According to the present invention, there is provided an aqueous hair cosmetic composition comprising:-

(A) 0.01-5 %wt polyethylene glycol having an average molecular weight of 500,000 or more,
(B) 0.1-30 %wt oil or fat, and
(C) 0.1-30 %wt nonionic surfactant.

Polyethylene glycols which are useful as component (A) in this invention are those capable of providing extendibility and smoothness when blended with nonionic surfactants and oils or fats while maintaining a good rinsing capability. Average molecular weight of the polyethylene glycol is 500,000 or more, and preferably 1,000,000 to 8,000,000. If less than 500,000, the mentioned requirements cannot be fully met. The amount of component (A) to be incorporated into the present hair cosmetic composition is 0.01 to 5% by weight, and more preferably 0.05 to 1% by weight of the total composition.

The terms "oil" and "fat" refer to liquid oil and solid fat. Oils and fats which are useful as component (B) in this invention are not limited so far as they are selected from those generally used for the preparation of hair cosmetic compositions. Examples of such oils and fats include higher alcohols having a linear or branched alkyl or alkenyl group; hydrocarbons such as liquid paraffin, vaseline and solid paraffin; lanolin derivatives such as liquid lanolin and lanolin fatty acid; silicones such as dimethylpolysiloxane and polyether-modified silicones; higher fatty acid esters, higher fatty acids and long-chain amidoamines having an alkyl or alkenyl group; animal or vegetable oils and fats such as mink oil and olive oil. Among them, especially preferred are monoglycerides derived from a saturated or unsaturated, linear or branched C12 - C24 fatty acid and higher alcohols having a linear or branched alkyl or alkenyl group of C12 - C26. More particularly, the following are mentioned: fatty acid monoglycerides such as oleic acid monoglyceride, palmitic acid monoglyceride, behenic acid monoglyceride, isostearic acid monoglyceride; and higher alcohols such as cetyl alcohol, stearyl alcohol, arachidic alcohol, behenyl alcohol, carnaubyl alcohol and ceryl alcohol. The amount of component (B) to be incorporated into the present hair cosmetic composition is from 0.1 to 30% by weight, and more preferably, from 1 to 10% by weight of the total composition.

Noionic surfactants which are useful as component (C) in this invention include: glyceryl fatty acid esters such as glyceryl monostearate, glyceryl distearate, diglyceryl monostearate, diglyceryl distearate, glyceryl monoisostearate, diglyceryl monoisostearate; polyoxyethylene fatty acid esters such as polyoxyethylene monostearate; polyoxyethylene-added hydrogenated castor oil; polyoxyethylene glyceryl fatty acid esters; polyoxyethylene alkyl ethers such as polyoxyethylene lauryl ether; polyoxyethylene alkyl phenyl ethers such as polyoxyethylene nonyl phenyl ether; sorbitan fatty acid esters such as sorbitan monolaurate; polyoxyethylene sorbitan fatty acid esters; glyceryl ethers such as monoisostearyl glyceryl ether; alkyl alkanol amides such as palmae oil fatty acid diethanol amide and isostearyl diethanol amide; trimethylols such as isostearyl trimethylolamide and trimethylolisoheptadecane; alkyl saccharides such as octyl glucoside, nonylglucoside, decyl glucoside, decyl maltoside and isostearyl glucoside; N-alkyl sugar amides such as N-lauryl gluconamide, N-lauryl maltobionamide and N-lauryl lactobionamide; sucrose fatty acid esters such as sucrose palmae oil fatty acid ester, sucrose laurate and sucrose stearate; maltitol fatty acid esters such as maltitol monolaurate and maltitol monostearate. The amount of component (C) to be incorporated into the present hair cosmetic composition is 0.1 to 30% by weight, more preferably, 1 to 10% by weight of the total composition.

The hair cosmetic composition of this invention, as they do not contain substantial amounts of a cationic surfactant, provide remarkable extendibility and smoothness upon use without giving sticky or greasy sensation to users. They, however, may optionally contain a small amount (2% by weight or less, and should not irritate the scalp or skin) of a cationic surfactant for the purpose of obtaining antistatic effect, etc.

Examples of cationic surfactants which are optionaly used in this invention include quaternary ammonium salts of the following formula

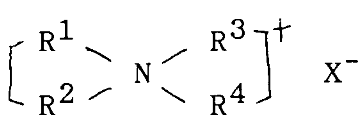 (I)

wherein one or two of $R^1$ to $R^4$ groups represent a C8 - C28 linear or branched alkyl or hydroxyalkyl group or a group wherein $R^5$ is a C8 - C28 alkyl or hydroxyalkyl group and r is a number of 1 to 10; the remaining $R^1$ to $R^4$ groups represent a benzyl group or a C1 - C3 alkyl or hydroxyalkyl group; and $X^-$ represents a halogen ion or an organic anion.

The hair cosmetic compositions of this invention can optionally contain other compatible ingredients which are generally incorporated into cosmetic compositions, drugs, foods, etc. Examples of such optional ingredients are pharmaceutical agents such as water, antidandruffs, germicides, and vitamins; antiseptics such as paraben; viscosity modifiers such as perfluoropolyethers and water-soluble polymers; colorants such as dyes and pigments; conditioning agents such as cationic polymers; pearling agents such as glycol esters; hair setting polymers such as acrylic resins; various compounded perfumes; and ingredients listed in "Encyclopedia of Conditioning Rinse Ingredients" (Micelle Press, 1987). They can be used in such an amount that will not impede the effects of this invention.

The hair cosmetic compositions of this invention can take any forms, among which emulsion, more specifically O/W emulsion, is preferred.

The hair cosmetic compositions of this invention are prepared according to known methods using the mentioned ingredients. The compositions of this invention are suitable for formulating hair rinses, hair

conditioners, hair treatments, and so on, so far as the final products are used in such a manner that they are directly applied to the shampooed hair while wet, and then rinsed off.

This invention will now be explained in more detail by reference to certain specific examples which are provided herein for the purpose of illustration only and are not intended to be limiting unless otherwise specified.

Example 1

Hair rinse compositions as shown in Table 1, which don't contain a cationic surfactant were prepared, and their rinsing performance was evaluated. The results are also shown in Table 1 where the ingredients are identified.

Process of preparation

Into ingredient (7) which was heated at 70 C were added ingredients (2), (3), (4) and (5) which were previously blended to dissolve at 70 C, and mixed to emulsify. To the obtained emulsion, ingredients (1) to (6) were added and cooled while stirring to obtain a hair rinse composition.

Test Method

Sensual Evaluation

20 g (15 cm long) of a virgin hair tress of a Japanese woman, which had never undergone cold-perming or bleaching, was provided for the test. The hair tress was applied with 2 g of a hair rinse composition, rinsed under running water for 30 seconds, towel-dried and then dried with a drier. Softness and smoothness of the hair were sensually evaluated with the following standard:

AA : excellent
A : good
B : so-so
C : bad

Combing force

Combing force of the hair tress applied with the hair rinse composition was measured as was (water content: about 0.7 g /g hair) or after dried with a drying apparatus by the use of a strain gauge. The measurement was repeated 20 times in total in a thermostat (20 C, 65% RH) and a mean value (g) was calculated to give a combing force.

Table 1

| | | Comparative Products | | Invention Product |
|---|---|---|---|---|
| | | 1 | 2 | 1 |
| (1) Polyethylene glycol | | | | |
| (M.W.= 2,000,000) | | - | 0.2 | 0.2 |
| (M.W.= 6,000) | | 0.2 | - | - |
| (2) Cetyl alcohol | | 5.0 | - | 5.0 |
| (3) Palmae oil fatty acid diethanolamide | | 4.0 | 4.0 | 4.0 |
| (4) Polyoxyethylene cetyl ether | | 4.0 | 4.0 | 4.0 |
| (5) Glycerin | | 5.0 | 5.0 | 5.0 |
| (6) Hydroxyethylcellulose | | 1.0 | 1.0 | 1.0 |
| (7) Water | | balance | → | → |
| Combing force (g) | When applied to the hair | 311 | 296 | 230 |
| | When dried | 161 | 190 | 140 |
| Smoothness | | C | C | AA |
| Softness | | C | C | A |

Example 2

Low-concentration cationic surfactants as shown in Table 2 were prepared according to a known method. The resulted hair rinse compositions all exhibited good extendibility upon application, excellent smoothness and softness, and agreeable sensation.

Table 2

| | Invention Products | | | |
|---|---|---|---|---|
| | 2 | 3 | 4 | 5 |
| Polyethylene glycol (M.W.= 2,000,000)*1 | 0.2 | 0.3 | - | - |
| Polyethylene glycol (M.W.= 4,000,000)*2 | - | - | 0.1 | 0.2 |
| Cetanol | 2.0 | 2.0 | 2.0 | 2.0 |
| Polyether-modified silicone*3 | 0.5 | - | - | - |
| Amino-modified silicone emulsion*4 | - | 0.5 | - | - |
| Stearyl trimethyl ammonium chloride | 0.6 | 0.6 | 0.6 | 0.6 |
| Vaseline | - | - | 0.4 | 0.4 |
| Self-emulsified glycerin monostearate | 0.2 | 0.2 | - | - |
| Polyoxyethylene oleyl ether (5 E.O.) | 0.3 | 0.3 | 0.3 | 0.3 |
| Isostearyl trimethilolamide | 1.5 | - | 1.5 | - |
| Isostearyl diethanolamide | - | 2.0 | - | 2.0 |
| Propylene glycol | 5.0 | 5.0 | 5.0 | 5.0 |
| Hydroxyethylcellulose | 0.6 | 0.6 | 0.6 | 0.6 |
| Antiseptic | suitable amount | → | → | → |
| Colorant | suitable amount | → | → | → |
| Perfume | suitable amount | → | → | → |
| Purified water | balance | → | → | → |

*1) POLYOX WSR N-60K (Union Carbide Inc.)
*2) POLYOX WSR-301 (Union Carbide Inc.)
*3) KF351A (Shin-etsu Kagaku Kogyo K.K.)
*4) SM8702C (Toray - Dowcorning Co.)

Example 3

A hair treatment composition of the following formula was prepared:

| | |
|---|---|
| Polyethylene glycol (M.W.=4,000,000) | 0.2 (wt.%) |
| Cetanol | 5.0 |
| Dimethyl polysiloxane (1000cs) | 1.0 |
| Vaseline | 0.5 |
| Dialkyl dimethyl ammonium chloride*1 | 0.4 |
| Stearyl trimethyl ammonium chloride | 0.4 |
| Palmae oil fatty acid diethanolamide | 4.0 |
| Polyoxyethylene cetyl ether (5 E.O.) | 0.5 |
| Glycerin | 5.0 |
| Hydroxyethylcellulose | 0.8 |
| Methylparaben | 0.2 |
| Perfume | 0.4 |
| Water | balance |

*1) Branched quaternary ammonium salt derived from a commercially available oxo-alcohol (C12 - C15) which is an equi-amount mixture of Dovanol 23 and Dovanol 45 (Mitsubishi Yuka K.K.), with a branching ratio of 20%.

The composition had remarkable extendibility upon use providing little greasy feel of the wet hair and dry hair, excellent smoothness and a good emulsion stability.

## Claims

1. An aqueous hair cosmetic composition comprising:-
   (A) 0.01-5 %wt polyethylene glycol having an average molecular weight of 500,000 or more,
   (B) 0.1-30 %wt oil or fat, and
   (C) 0.1-30 %wt nonionic surfactant.

2. An aqueous hair cosmetic composition according to claim 1 wherein said component (A) is a polyethylene glycol having an average molecular weight of 1,000,000 - 8,000,000.

3. An aqueous hair cosmetic composition according to claim 1 or claim 2 wherein said component (B) is a higher alcohol having a linear or branched alkyl or alkenyl group having 12-26 carbon atoms or a monoglyceride which is derived from a saturated or unsaturated linear or branched fatty acid having 12 to 24 carbon atoms.

**4.** An aqueous hair cosmetic composition according to any of the preceding claims, further comprising 2 %wt by weight or less of a cationic surfactant.

**5.** An aqueous hair cosmetic composition according to any of the preceding claims in emulsified form.

**6.** A method of treating hair which comprises applying a hair cosmetic composition in accordance with any of the preceding claims to shampooed hair, and thereafter rinsing the hair.

**7.** A method of manufacturing an aqueous hair cosmetic composition as claimed in any of claims 1-5, characterised by combining:
(A) 0.01-5 %wt polyethylene glycol having an average molecular weight of 500,000 or more,
(B) 0.1-30 %wt oil or fat, and
(C) 0.1-30 %wt nonionic surfactant
in an aqueous medium.

**Patentansprüche**

**1.** Wäßrige haarkosmetische Zusammensetzung umfassend:
(A) 0,01-5 Gew.% Polyethylenglykol mit einem durchschnittlichen Molekulargewicht von 500 000 oder mehr,
(B) 0,1-30 Gew.% Öl oder Fett und
(C) 0,1-30 Gew.% nichtionisches oberflächenaktives Mittel.

**2.** Wäßrige haarkosmetische Zusammensetzung nach Anspruch 1, worin die Komponente (A) ein Polyethylenglykol mit einem durchschnittlichen Molekulargewicht von 1 000 000 - 8 000 000 ist.

**3.** Wäßrige haarkosmetische Zusammensetzung nach Anspruch 1 oder Anspruch 2, worin die Komponente (B) ein höherer Alkohol mit einer linearen oder verzweigten Alkyl- oder Alkenyl-Gruppe mit 12-26 Kohlenstoffatomen oder ein Monoglycerid ist, das von einer gesättigten oder ungesättigten, linearen oder verzweigten Fettsäure mit 12 bis 24 Kohlenstoffatomen stammt.

**4.** Wäßrige haarkosmetische Zusammensetzung nach einem der vorhergehenden Ansprüche, die außerdem 2 Gew.% oder weniger eines kationischen oberflächenaktiven Mittels umfaßt.

**5.** Wäßrige haarkosmetische Zusammensetzung nach einem der vorhergehenden Ansprüche in emulgierter Form.

**6.** Verfahren zur Haarbehandlung, das das Auftragen einer haarkosmetischen Zusammensetzung nach einem der vorhergehenden Ansprüche auf schampuniertes Haar und das anschließende Spülen des Haars umfaßt.

**7.** Verfahren zum Herstellen einer wäßrigen haarkosmetischen Zusammensetzung, wie sie in einem der Ansprüche 1-5 beansprucht wird, gekennzeichnet durch Kombinieren von:
(A) 0,01-5 Gew.% Polyethylenglykol mit einem durchschnittlichen Molekulargewicht von 500 000 oder mehr,
(B) 0,1-30 Gew.% Öl oder Fett und
(C) 0,1-30 Gew.% nichtionischem oberflächenaktiven Mittel in einem wäßrigen Medium.

**Revendications**

**1.** Une compostion cosmétique aqueuse pour les cheveux comprenant :
(A) 0,01-5 % en poids de polyéthylène-glycol présentant un poids moléculaire moyen de 500.000 ou plus,
(B) 0,1-30 % en poids d'huile ou de corps gras, et
(C) 0,1-30 % en poids d'agents tensio-actifs nonioniques

**2.** Une composition cosmétique aqueuse pour les cheveux conformément à la revendication 1, dans laquelle ledit composant (A) est un polyéthylène-glycol présentant un poids moléculaire moyen de 1

000 000 - 8 000 000.

**3.** Une composition cosmétique aqueuse pour les cheveux conformément à la revendication 1 ou la revendication 2, dans laquelle ledit composant (B) est un alcool élevé présentant un groupe alcoyle ou alcényle ramifié ou linéaire présentant 12-26 atomes de carbone ou un monoglycéride qui est dérivé d'un acide gras linéaire ou ramifié saturé ou insaturé ayant 12 à 24 atomes de carbone.

**4.** Une composition cosmétique aqueuse pour les cheveux conformément à l'une quelconque des revendications précédentes, comprenant en outre 2 % en poids ou moins d'un agent tensionactif cationique.

**5.** Une composition cosmétique aqueuse pour les cheveux conformément à l'une quelconque des revendications précédentes, sous forme d'émulsion.

**6.** Un procédé de traitement capillaire qui comprend l'application d'une composition cosmétique pour les cheveux selon l'une quelconque des revendications précédentes sur le cheveu shampouiné, et ensuite le rinçage du cheveu.

**7.** Un procédé de fabrication d'une composiion cosmétique aqueuse pour les cheveux comme revendiqué dans l'une quelconque des revendications 1-5, caractérisé par la combinaison :

(A) 0,01-5 % en poids de polyéthylène-glycol présentant un poids moléculaire moyen de 500 000 ou plus,
(B) 0,1-30 % en poids d'huile ou de corps gras, et
(C) 0,1-30 %pds d'agents tensio-actifs nonioniques

dans un milieu aqueux.